# FASCICULE DE BREVET EUROPEEN

(11) **EP 1 324 739 B1**
(45) Date de publication et mention de la délivrance du brevet: **24.05.2006**
(21) Numéro de dépôt: 01976374.7
(22) Date de dépôt: 08.10.2001
(51) Int. Cl.: A61K 8/34

(54) **COMPOSITION COSMETIQUE COMPRENANT UN LATEX ANIONIQUE, UN ETHER A DEUX CHAINES GRASSES ET UN MELANGE D'ALCOOLS GRAS**
KOSMETISCHE ZUSAMMENSETZUNG ENTHALTEND EIN ANIONISCH LATEX, ETHER MIT ZWEIT FETTKETTEN UND EINER FETTALKOHOL MISCHUNG
COSMETIC COMPOSITION COMPRISING AN ANIONIC LATEX, AN ETHER HAVING TWO FATTY CHAINS AND A MIXTURE OF FATTY ALCOHOLS

(30) Priorité: 13.10.2000 FR 0013172
(43) Date de publication de la demande: 09.07.2003
(73) Titulaire: L'OREAL, 75008 Paris (FR)
(72) Inventeur: MAUBRU, Mireille, F-78400 Chatou (FR); QEAUQUEY, Bernard, F-92110 Clichy (FR)
(74) Mandataire: Casalonga, Axel
(86) Numéro de dépôt international: PCT/FR2001/003090
(87) Numéro de publication internationale: WO 2002/030370

(56) Documents cités:
- EP-A- 0 976 393
- WO-A-99/09944
- DE-A- 19 927 653

## Description

La présente invention est relative à une composition cosmétique, comprenant dans un milieu cosmétiquement acceptable, au moins un agent tensioactif, au moins un latex anionique, au moins un éther à deux chaînes grasses et un mélange d'alcools gras linéaires et saturés, à un procédé de traitement cosmétique des matières kératiniques et à une utilisation de ladite composition cosmétique comme shampoing.

Les associations connues, dans des shampoings nacrés, de latex anioniques et de distéarate de glycol conduisent à des propriétés cosmétiques qui ne sont pas totalement satisfaisantes, notamment sur cheveux mouillés.

La demanderesse a trouvé de manière surprenante que l'utilisation particulière d'un éther à deux chaînes grasses, solide à température ambiante, en association avec un mélange d'alcools gras linéaires et saturés à longue chaîne contenant au moins 50 % en poids d'un alcool qui comporte 22 atomes de carbone, et des latex anioniques, dans un milieu cosmétiquement acceptable, améliorait sensiblement les propriétés cosmétiques sur les cheveux mouillés, et en particulier la légèreté.

La présente invention a donc pour objet une composition cosmétique comprenant, dans un milieu cosmétiquement acceptable, au moins un agent tensioactif, au moins un latex anionique, au moins un éther à deux chaînes grasses et un mélange d'alcools gras linéaires et saturés à longue chaîne contenant au moins 50 % en poids d'un alcool qui comporte 22 atomes de carbone.

Par milieu cosmétiquement acceptable, on entend un milieu compatible avec toutes les matières kératiniques telles que la peau, les cheveux, les ongles, les cils et sourcils, les lèvres et toute autre zone du corps et du visage, mais aussi d'odeur, d'aspect et de toucher agréables.

Un autre objet de l'invention consiste en un procédé de traitement cosmétique des matières kératiniques mettant en oeuvre la composition susvisée.

L'invention a encore pour objet une utilisation de la composition selon l'invention comme shampoing.

D'autres objets, caractéristiques, aspects et avantages de l'invention apparaîtront encore plus clairement à la lecture de la description et des divers exemples qui suivent.

Selon l'invention, la composition cosmétique comprend, dans un milieu cosmétiquement acceptable, au moins un agent tensioactif, au moins un latex anionique, au moins un éther à deux chaînes grasses et un mélange d'alcools gras linéaires et saturés à longue chaîne contenant au moins 50 % en poids d'un alcool qui comporte 22 atomes de carbone.

Les éthers à deux chaînes grasses convenant dans la présente invention sont notamment choisis parmi les éthers à deux chaînes grasses, solides à une température inférieure ou égale à environ 30°C, répondant à la formule suivante :

R-O-R' (I)

dans laquelle :
R et R', identiques ou différents, désignent un groupe hydrocarboné, saturé ou insaturé, linéaire ou ramifié, comportant de 12 à 30 atomes de carbone et de préférence de 14 à 24 atomes de carbone, R et R' étant choisis de façon telle que le composé de formule (I) soit solide à une température inférieure ou égale à 30° C environ. De préférence, R et R' sont identiques et désignent un groupe alkyle tel que stéaryle.

Les éthers à deux chaînes grasses utilisables selon l'invention sont insolubles dans les compositions cosmétiques, et peuvent être préparés selon le procédé décrit dans la demande de brevet DE 41 27 230.

On utilise notamment dans le cadre de la présente invention, le distéaryléther vendu sous la dénomination CUTINA® STE par la société COGNIS.

Selon l'invention, l'éther à deux chaînes grasses peut représenter de 0,5 % à 15 % en poids, de préférence de 0,5 % à 5 % en poids, et encore plus préférentiellement de 1 % à 3 % en poids, par rapport au poids total de la composition finale.

Le mélange d'alcools gras linéaires et saturés à longue chaîne contenant au moins 50 % en poids d'un alcool qui comporte 22 atomes de carbone, contient plus particulièrement au moins 70 % en poids d'alcool en C₂₂ par rapport au poids total du mélange.

De manière générale, le mélange d'alcools gras linéaires et saturés à longue chaîne contient des alcools gras en C₁₆ à C₂₄. Les alcools gras en C₁₆ et C₂₄ représentent chacun généralement moins de 2 % en poids, les alcools en C₁₈ moins de 10 % en poids du poids total du mélange.

A titre d'exemples de tels alcools gras, on peut notamment citer le produit commercialisé sous la dénomination NAFOL® 1822 C par la société CONDEA qui contient environ 0,5 % d'alcool en C₁₆, 4-6 % d'alcool en C₁₈, 15-19 % d'alcool en C₂₀, 74-78 % d'alcool en C₂₂ et environ 1,5 % d'alcool en C₂₄, ou le produit commercialisé sous la dénomination NAFOL® 2298 par la société CONDEA qui contient 98 % en poids d'alcool en C₂₂.

Selon l'invention, le mélange d'alcools gras linéaires et saturés à longue chaîne, contenant au moins 50 % en poids d'alcool qui comporte 22 atomes de carbone, peut représenter de 0,5 % à 15 % en poids, de préférence de 0,5 % à 5 % en poids, et encore plus préférentiellement de 1 % à 3 % en poids, par rapport au poids total de la composition cosmétique.

Le rapport éther à deux chaînes grasses/alcools gras en C₂₂ est généralement compris entre 0,2 et 8 et de préférence entre 0,3 et 5.

Par latex anionique, on entend une dispersion colloïdale de particules de polymère anionique dans une phase liquide aqueuse ou organique.

Les latex utilisés selon l'invention, sont ceux contenant des groupements fonctionnels anioniques qui sont apportés soit par polymérisation ou copolymérisation en émulsion ou en suspension de monomères anioniques, par les procédés habituels connus de l'homme de métier, soit par combinaison du radical libre issu de l'amorceur de réaction sur le monomère ou les monomères considérés lors de l'initiation de la polymérisation desdits monomères, ou bien par juxtaposition de ces deux procédés de synthèse, ou bien encore par introduction de groupements terminaux à travers une réaction de transfert de chaînes dans le second procédé mettant en oeuvre un amorceur de réaction.

Le procédé mettant en oeuvre des amorceurs de réaction est décrit en particulier dans l'article de R. M. FITCH, "Preparation and characterization of charge stabilised polymer colloids" dans "Polyelectrolytes and their applications", 51-69 par D. REIDER publishing company.

Les latex utilisés plus particulièrement dans les compositions selon l'invention résultent de la polymérisation d'un ou plusieurs des monomères ionogènes suivants : les acides acrylique, méthacrylique, maléïque, crotonique, itaconique, para-styrènesulfonique, vinylsulfonique, 2-méthacryloyloxyéthylsulfonique et 2-acrylamido-2-méthylpropylsulfonique, seuls ou en mélange avec différents monomères tels que le styrène, le butadiène, l'éthylène, le propylène, l'isoprène, l'isobutène, le vinyltoluène, le propionate de vinyle, l'alcool vinylique, l'acrylonitrile, le chloroprène, le chlorure de vinyle, le chlorure de vinylidène, l'acétate de vinyle, les uréthannes, l'éther vinylique, la vinylpyrrolidone, le vinylimidazole, les esters des acides acrylique, méthacrylique, vinylacétique, maléïque, crotonique ou itaconique.

Les latex obtenus selon le second procédé ci-dessus résultent de l'utilisation d'amorceurs choisis parmi les systèmes oxydo-réducteurs, les peroxydes, les perphosphates, les percarbonates, les persulfates, les acides organiques peroxydés tels que, par exemple, l'acide peracétique, le mélange persulfate-bisulfite-fer.

Les monomères fonctionnels utilisés dans le cas de réaction de transfert de chaînes sont choisis parmi les thioacides organiques tels que, par exemple, l'acide mercaptoacétique.

Comme latex anionique particulièrement préféré de l'invention, on peut utiliser, par exemple, une dispersion aqueuse comprenant un copolymère acrylique formé de (a) environ 35 à 74 % en poids d'un acrylate d'alkyle, (b) environ 25 à 65 % de méthacrylate d'alkyle et (c) environ 1 à 15 % d'un ou plusieurs acides carboxyliques éthyléniques ou leurs sels, ayant de 3 à 5 atomes de carbone, les groupes alkyle comportant de 1 à 5 atomes de carbone, ces pourcentages étant exprimés en poids par rapport au poids total de copolymère.

L'acrylate d'alkyle est de préférence choisi parmi l'acrylate de méthyle, l'acrylate d'éthyle, l'acrylate de propyle et l'acrylate de butyle. L'acrylate d'éthyle est particulièrement préféré.

La concentration d'acrylate d'alkyle est de préférence comprise entre 40 et 70 % en poids et plus particulièrement entre 50 et 60 % en poids par rapport au poids total du copolymère.

Le méthacrylate d'alkyle est de préférence choisi parmi le méthacrylate de méthyle, le méthacrylate d'éthyle, le méthacrylate de propyle et le méthacrylate de butyle. Le méthacrylate de méthyle est particulièrement préféré.

La concentration de méthacrylate d'alkyle est de préférence comprise entre 30 et 50 % en poids et plus particulièrement entre 30 et 40 % en poids par rapport au poids total du copolymère.

Les acides carboxyliques éthyléniques préférés sont l'acide acrylique, l'acide méthacrylique, l'acide crotonique, l'acide itaconique ou leurs mélanges. L'acide acrylique et l'acide méthacrylique sont particulièrement préférés. Selon l'invention, il est aussi possible de mettre en oeuvre des sels de ces acides carboxyliques.

La concentration d'acides carboxyliques éthy.léniques, ou de leurs sels, est de préférence comprise entre 5 et 15 % en poids et plus particulièrement entre 8 et 12 % en poids par rapport au poids total du copolymère. Dans un mode de réalisation particulièrement préféré de l'invention, l'acide acrylique est utilisé avec l'acide méthacrylique, chacun dans une concentration comprise entre 2 et 10 % en poids, la concentration totale de ces deux acides n'excédant pas 15 % en poids du poids total du copolymère.

Le copolymère peut également contenir des faibles quantités, c'est-à-dire moins de 10 %, de préférence moins de 5 % et plus particulièrement moins de 2%, d'un monomère polymérisable autre que ceux mentionnés ci-dessus.

Généralement, la dispersion contient au moins 0,5 % de tensioactif permettant la mise en dispersion et le maintien en dispersion du polymère insoluble. Selon l'invention, on peut utiliser tout type de tensioactif, mais de préférence un tensioactif non ionique.

La taille moyenne des particules du copolymère dans la dispersion est de préférence comprise entre 0,1 et 1 micromètre.

Selon un mode particulièrement préféré de mise en oeuvre de l'invention, on utilise un copolymère comprenant de 50 à 60 % en poids d'acrylate d'éthyle, de 30 à 40 % en poids de méthacrylate de méthyle, de 2 à 10% en poids d'acide acrylique, de 2 à 10% en poids d'acide méthacrylique, la concentration totale d'acides acrylique et méthacrylique n'excédant pas 15% en poids par rapport au poids total du copolymère acrylique.

Un tel copolymère est par exemple décrit dans la demande de brevet EP-A-590604.

Une dispersion aqueuse du copolymère acrylique décrit ci-dessus, comprenant 25 % en poids d'un copolymère acrylate d'éthyle/méthacrylate de méthyle/acide méthacrylique/acide acrylique ayant une température de transition vitreuse d'environ 30°C, est vendu notamment sous la dénomination commerciale AMERHOLD® DR-25 par la société AMERCHOL.

Les latex anioniques peuvent être utilisés en une quantité comprise entre 0,05 et 20 % en poids de matières actives, de préférence entre 0,15 et 5 % en poids de matières actives par rapport au poids total de la composition.

Les agents tensioactifs convenant dans la présente invention sont notamment choisis parmi les tensioactifs anioniques, amphotères, non-ioniques, cationiques bien connus dans la technique et tels que décrits ci-dessous, et leurs mélanges.

Comme tensioactifs anioniques utilisables dans la présente invention, on peut notamment mentionner les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéridesulfates ; les alkylsulfonates, les alkylamidesulfonates, les alkylarylsulfonates, les α-oléfine-sulfonates, les paraffine-sulfonates ; les alkylsulfosuccinates, les alkyléthersulfosuccinates, les alkylamidesulfosuccinates ; les alkylsulfoacétates ; les acylsarcosinates ; et les acylglutamates, les groupes alkyle et acyle de tous ces composés comportant de 6 à 24 atomes de carbone et le groupe aryle désignant de préférence un groupe phényle ou benzyle. On peut également utiliser les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les alkylsulfosuccinamates, les acyliséthionates et les N-acyltaurates, le groupe alkyle ou acyle de tous ces composés comportant de 12 à 20 atomes de carbone. Parmi les tensioactifs anioniques encore utilisables, on peut également citer les acyllactylates dont le groupe acyle comporte de 8 à 20 atomes de carbone.

En outre, on peut encore citer les acides d'alkyl-D-galactoside uroniques et leurs sels ainsi que les acides alkyl(C₆-C₂₄)éther-carboxyliques polyoxyalkylénés, les acides alkyl(C₆-C₂₄)aryl(C₆₋C₂₄)éther-carboxyliques polyoxyalkylénés, les acides alkyl(C₆₋C₂₄)amidoéther carboxyliques polyoxyalkylénés et leurs sels, en particulier ceux comportant de 2 à 50 groupements oxyde d'éthylène, et leurs mélanges.

Les tensioactifs anioniques tels que décrits ci-dessus, peuvent être utilisés seuls ou en mélange. On utilise de préférence les alkylsulfates, les alkyléthersulfates et les alkyléthercarboxylates, et leurs mélanges, en particulier sous forme de leurs sels de métaux alcalins ou alcalino-terreux, d'ammonium, d'amine ou d'aminoalcool.

Le tensioactif anionique préférentiellement utilisé dans la présente invention est un lauryléthersulfate de sodium.

Les tensioactifs amphotères convenant dans l'invention sont, par exemple, des dérivés d'amines aliphatiques secondaires ou tertiaires, dans lesquels le groupe aliphatique est une chaîne linéaire ou ramifiée comportant de 8 à 22 atomes de carbone et contenant, au moins un groupe anionique hydrosolubilisant tel que, par exemple, un groupe carboxylate, sulfonate, sulfate, phosphate ou phosphonate ; on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes ; et leurs mélanges.

Parmi les dérivés d'amines, on peut citer les produits commercialisés sous la dénomination MIRANOL® , tels que décrits dans les brevets US-2 528 378 et US-2 781 354 et classés dans le dictionnaire CTFA, 3^{ème} édition, 1982, sous les dénominations Amphocarboxyglycinate et Amphocarboxypropionate de structures respectives (II) et (III) :

R₁-CONHCH₂CH₂-N⁺(R₂)(R₃)(CH₂COO⁻) (II)

dans laquelle :
R₁ représente un groupe alkyle dérivé d'un acide R,-COOH présent dans l'huile de coprah hydrolysée, un groupe heptyle, nonyle ou undécyle,
R₂ représente un groupe bêta-hydroxyéthyle, et
R₃ représente un groupe carboxyméthyle; et

   R_{1'}-CONHCH₂CH₂-N(B)(C) (III)
dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
R₁, représente un groupe alkyle d'un acide R_{g}-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé sous la dénomination commerciale MIRANOL® C2M concentré par la société RHODIA.

Parmi les tensioactifs amphotères, on utilise de préférence les alkyl(C₈-C₂₀)bétaïnes, les alkyl(C₈-C₂₀)amidoalkyl(C₆-C₈)bétaïnes, les alkylamphodiacétates et leurs mélanges.

Lés tensioactifs non-ioniques convenant dans l'invention sont des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi les alcools, les alpha-diols, les alkyl(C,-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)-aminopropylmorpholine ; et leurs mélanges.

Parmi les tensioactifs non-ioniques cités ci-dessus, on utilise de préférence les alkyl(C₆-C₂₄)polyglucosides.

La composition selon l'invention peut également comprendre un ou plusieurs tensioactifs cationiques bien connus en soi, tels que les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyl-trialkylammonium, de trialkylbenzylammonium, de trialkylhydroxy-alkylammonium ou d'alkylpyridinium ; les dérivés d'imidazoline ; ou les oxydes d'amines à caractère cationique.

Les agents tensioactifs, tels que décrits ci-dessus, sont notamment utilisés en une quantité totale comprise entre 4 et 30 % en poids, de préférence entre 6 et 25 % en poids, et plus préférentiellement entre 8 et 22 % en poids par rapport au poids total de la composition.

Le milieu cosmétiquement acceptable peut être constitué par de l'eau ou par un mélange d'eau et d'un solvant cosmétiquement acceptable tel qu'un alcool inférieur en C₁-C₄, par exemple l'éthanol, l'isopropanol, le tertio-butanol, le n-butanol ; les alkylèneglycols comme le propylèneglycol, les éthers de glycol ; les alcanes en C₅₋C₁₀ ; l'acétone, la méthyléthylcétone ; les acétates d'alkyle en C₁-C₄ comme l'acétate de méthyle, l'acétate d'éthyle, l'acétate de butyle ; le diméthoxyéthane, le diéthoxyéthane ; et leurs mélanges.

Le pH des compositions de l'invention est compris entre 3 et 8, de préférence entre 4 et 7.

Les compositions selon l'invention peuvent également contenir des additifs classiques bien connus dans la technique, tels que des polymères cationiques, non ioniques ou amphotères, des polymères anioniques autres que ceux de l'invention, des silicones volatiles ou non, modifiées ou non, des épaississants polymériques naturels ou synthétiques, anioniques, amphotères, zwittérioniques, non-ioniques ou cationiques, associatifs ou non, des épaississants non polymériques comme des acides ou des électrolytes, des opacifiants, des parfums; des huiles minérales, végétales et/ou synthétiques, des esters d'acides gras ou de polyéthylèneglycols, des colorants, des particules organiques ou minérales, des conservateurs, des agents de stabilisation du pH.

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Une classe d'additifs privilégiés est constituée par les polymères coiffants, et en particulier les polymères à squelette polysiloxane greffés par des motifs hydrocarbonés, et de préférence acryliques.

Une autre classe est constituée par les polymères cationiques à motifs vinyllactames.

Les compositions cosmétiques selon l'invention peuvent se présenter sous forme de liquides fluides ou épaissis, de gels, de crèmes, de mousses, d'émulsions simples ou d'émulsions multiples.

Elles peuvent être utilisées, par exemple, comme shampoings, soins rincés, masques de soin profond, gels douches, lotions ou crèmes de traitement du cuir chevelu.

La présente invention concerne également un procédé de traitement cosmétique des matières kératiniques qui consiste à appliquer une quantité efficace d'uné composition cosmétique telle que décrite ci-dessus, sur les matières kératiniques, à effectuer un éventuel rinçage après un éventuel temps de pose.

Selon un mode de réalisation préféré de l'invention, la composition peut être utilisée comme shampoing.

Les exemples suivants illustrent la présente invention et ne doivent être considérés en aucune manière comme limitant l'invention.

### Exemples

On a préparé deux compositions A et B de shampoing selon l'invention à partir des ingrédients suivants :

| Composition | A | B |
|---|---|---|
| Lauryléthersulfate de sodium (C12/C14 à 70/30) à 2,2 moles d'oxyde d'éthylène | 10,5 g M.A | 10,5 g M.A |
| Tensioactif amphotère dérivé de l'imidazoline, vendu sous la dénomination Miranol® C2M par la société Rhodia (40 % M.A.) | 1,2 g M.A | 1,2 g M.A |
| Alkyl(C₈/C₁₆) polyglycoside (1,4) en solution aqueuse à 53 % vendu sous la dénomination PLANTACARE® 818 UP par la société COGNIS. | 8,0 g M.A | 8,0 g M.A |
| Alcool laurique oxyéthyléné (2,5 moles d'oxyde d'éthylène) | 0,75 g | 0,75 g |
| Copolymère vinylpyrrolidone/chlorure de méthylvinylimidazolium (5/95) en solution aqueuse à 40 % | 1,3 g | 1,3 g |
| Copolymère acide méthacrylique/acide acrylique/ acrylate d'éthyle/méthacrylate de méthyle en dispersion aqueuse à 25 %, commercialisé sous la dénomination Amerhold® DR 25 par la société Amerchol | 0,2 g M.A. | 0,2 g M.A |
| Acide polyacrylique réticulé | 0,2 g | 0,2 g |
| Distéarate de glycol | 3,0 g | - |
| Distéaryléther | - | 1,5 g |
| Mélange d'alcools gras à 76 % d'alcool béhénique commercialisé sous la dénomination Nafol® 1822C par la société Condéa | - | 1,5 g. |
| Acide citrique qsp | pH 5 | pH 5 |
| Chlorure de sodium | 1,5 g | 1,5 g |
| Parfum, conservateur | q.s. | q.s. |
| Eau déminéralisée qsp | 100,0 g | 100,0 g |

| | | |
|---|---|---|
| M.A. : matière active | | |

La composition B est une composition selon l'invention tandis que la composition A est une composition de l'art antérieur (comme celui décrit dans le document EP-A-0 976 393).

On applique les deux compositions sur les cheveux et on rince.

On observe sur cheveux mouillés un meilleur décollement des racines et un apport de légèreté avec la composition B selon l'invention.

## Revendications

1. Composition cosmétique **caractérisée en ce qu'**elle comprend, dans un milieu cosmétiquement acceptable, au moins un agent tensioactif, au moins un latex anionique, au moins un éther à deux chaînes grasses et un mélange d'alcools gras linéaires et saturés à longue chaîne contenant au moins 50 % en poids d'un alcool qui comporte 22 atomes de carbone.

2. Composition cosmétique selon la revendication 1, **caractérisée en ce que** l'éther à deux chaînes grasses est choisi parmi les éthers à deux chaînes grasses, solides à une température inférieure ou égale à environ 30°C, répondant à la formule suivante :
R-O-R' (I)
dans laquelle :
R et R', identiques ou différents, désignent un groupe hydrocarboné, saturé ou insaturé, linéaire ou ramifié, comportant de 12 à 30 atomes de carbone et de préférence de 14 à 24 atomes de carbone, R et R' étant choisis de façon telle que le composé de formule (I) soit solide à une température inférieure ou égale à 30° C environ

3. Composition cosmétique selon la revendication 1 ou 2, **caractérisée en ce que** l'éther à deux chaînes grasses est un distéaryléther.

4. Composition cosmétique selon l'une quelconque des revendications précédentes, **caractérisée en ce que** le mélange d'alcools gras contient au moins 50 % en poids d'un alcool qui comporte 22 atomes de carbone, par rapport au poids total du mélange.

5. Composition cosmétique selon l'une quelconque des revendications précédentes, **caractérisée en ce que** le latex anionique résulte de la polymérisation d'un ou plusieurs des monomères ionogènes suivants : les acides acrylique, méthacrylique, maléïque, crotonique, itaconique, para-styrènesulfonique, vinylsulfonique, 2-méthacryloyloxyéthylsulfonique et 2-acrylamido-2-méthylpropylsulfonique, seuls ou en mélange avec différents monomères tels que le styrène, le butadiène, l'éthylène, le propylène, l'isoprène, l'isobutène, le vinyltoluène, le propionate de vinyle, l'alcool vinylique, l'acrylonitrile, le chloroprène, le chlorure de vinyle, le chlorure de vinylidène, l'acétate de vinyle, les uréthannes, l'éther vinylique, la vinylpyrrolidone, le vinylimidazole, les esters des acides acrylique, méthacrylique, vinylacétique, maléïqué, crotonique ou itaconique.

6. Composition cosmétique selon l'une quelconque des revendications précédentes, **caractérisée en ce que** le latex anionique est une dispersion aqueuse comprenant un copolymère acrylique formé de (a) environ 35 à 74 % en poids d'un acrylate d'alkyle, (b) environ 25 à 65 % en poids de méthacrylate d'alkyle et (c) environ 1 à 15% en poids d'un ou plusieurs acides carboxyliques éthyléniques ou leurs sels ayant de 3 à 5 atomes de carbone , les groupes alkyle comportant de 1 à 5 atomes de carbone et ces pourcentages en poids étant exprimés par rapport au poids total du copolymère.

7. Composition cosmétique selon la revendication 6, **caractérisée en ce que** ledit copolymère comprend de 50 à 60 % en poids d'acrylate d'éthyle, de 30 à 40 % en poids de méthacrylate de méthyle, de 2 à 10 % en poids d'acide acrylique, de 2 à 10 % en poids d'acide méthacrylique, la concentration totale d'acides acrylique et méthacrylique n'excédant pas 15 % du poids total du copolymère.

8. Composition cosmétique selon l'une quelconque des revendications précédentes, **caractérisée en ce que** les agents tensioactifs sont choisis parmi les tensioactifs anioniques, amphotères, non ioniques, cationiques et leurs mélanges.

9. Composition cosmétique selon l'une quelconque des revendications précédentes, **caractérisée en ce que** l'éther à deux chaînes grasses est utilisé en une quantité de 0,5 % en poids à 15 %, en poids par rapport au poids total de la composition cosmétique.

10. Composition cosmétique selon l'une quelconque des revendications précédentes, **caractérisée en ce que** le mélange d'alcools gras linéaires et saturés à longue chaîne contenant au moins 50 % en poids d'un alcool qui comporte 22 atomes est utilisé en une quantité de 0,5 % en poids à 15 % en poids par rapport au poids total de la composition cosmétique.

11. Composition cosmétique selon l'une quelconque des revendications précédentes, **caractérisée en ce que** le latex anionique est utilisé en une quantité de 0,05 à 20 % en poids de matières actives par rapport au poids total de la composition cosmétique.

12. Composition cosmétique selon l'une quelconque des revendications précédentes, **caractérisée en ce que** les agents tensioactifs sont utilisés en une quantité totale de 4 % en poids à 30 % en poids par rapport au poids total de la composition cosmétique.

13. Composition cosmétique selon l'une quelconque des revendications précédentes, **caractérisée en ce qu'**elle contient en outre un polymère coiffant.

14. Composition cosmétique selon l'une quelconque des revendications précédentes, **caractérisée en ce qu'**elle contient en outre un polymère cationique.

15. Procédé de traitement cosmétique des matières kératiniques, **caractérisé en ce que** l'on applique une quantité efficace d'une composition cosmétique selon l'une quelconque des précédentes revendications sur les matières kératiniques, et **en ce que** l'on effectue un éventuel rinçage après un éventuel temps de pose.

16. Utilisation d'une composition cosmétique selon l'une quelconque des revendications 1 à 14, comme shampoing.

## Claims

1. Cosmetic composition, **characterized in that** it comprises, in a cosmetically acceptable medium, at least one surfactant, at least one anionic latex, at least one ether having two fatty chains and a mixture of long chain linear and saturated fatty alcohols containing at least 50% by weight of an alcohol which contains 22 carbon atoms.

2. Cosmetic composition according to Claim 1, **characterized in that** the ether having two fatty chains is chosen from ethers having two fatty chains, which are solid at a temperature of less than or equal to about 30°C, and which correspond to the following formula:
R-O-R' (I)
in which:
R and R', which are identical or different, denote a linear or branched, saturated or unsaturated hydrocarbon group containing from 12 to 30 carbon atoms, and preferably from 14 to 24 carbon atoms, R and R' being chosen such that the compound of formula (I) is solid at a temperature of less than or equal to about 30°C.

3. Cosmetic composition according to Claim 1 or 2, **characterized in that** the ether having two fatty chains is a distearyl ether.

4. Cosmetic composition according to any one of the preceding claims, **characterized in that** the mixture of fatty alcohols contains at least 50% by weight of an alcohol which contains 22 carbon atoms, relative to the total weight of the mixture.

5. Cosmetic composition according to any one of the preceding claims, **characterized in that** the anionic latex results from the polymerization of one or more of the following ionogenic monomers: acrylic, methacrylic, maleic, crotonic, itaconic, para-styrenesulphonic, vinylsulphonic, 2-methacryloyloxyethylsulphonic and 2-acrylamido-2-methylpropylsulphonic acids, alone or as a mixture with various monomers such as styrene, butadiene, ethylene, propylene, isoprene, isobutene, vinyltoluene, vinyl propionate, vinyl alcohol, acrylonitrile, chloroprene, vinyl chloride, vinylidene chloride, vinyl acetate, urethanes, vinyl ether, vinylpyrrolidone, vinylimidazole, esters of acrylic, methacrylic, vinylacetic, maleic, crotonic or itaconic acids.

6. Cosmetic composition according to any one of the preceding claims, **characterized in that** the anionic latex is an aqueous dispersion comprising an acrylic copolymer consisting of (a) about 35 to 74% by weight of an alkyl acrylate, (b) about 25 to 65% by weight of alkyl methacrylate and (c) about 1 to 15% by weight of one or more ethylenic carboxylic acids or their salts, having from 3 to 5 carbon atoms, alkyl groups containing from 1 to 5 carbon atoms, these percentages being expressed by weight relative to the total weight of the copolymer.

7. Cosmetic composition according to Claim 6, **characterized in that** said copolymer comprises from 50 to 60% by weight of ethyl acrylate, from 30 to 40% by weight of methyl methacrylate, from 2 to 10% by weight of acrylic acid, from 2 to 10% by weight of methacrylic acid, the total concentration of acrylic and methacrylic acids not exceeding 15% of the total weight of the copolymer.

8. Cosmetic composition according to any one of the preceding claims, **characterized in that** the surfactants are chosen from anionic, amphoteric, nonionic and cationic surfactants, and mixtures thereof.

9. Cosmetic composition according to any one of the preceding claims, **characterized in that** the ether having two fatty chains is used in a quantity of 0.5% by weight to 15% by weight relative to the total weight of the cosmetic composition.

10. Cosmetic composition according to any one of the preceding claims, **characterized in that** the mixture of long chain linear and saturated fatty alcohols containing at least 50% by weight of an alcohol which contains 22 atoms is used in a quantity of 0.5% by weight to 15% by weight relative to the total weight of the cosmetic composition.

11. Cosmetic composition according to any one of the preceding claims, **characterized in that** the anionic latex is used in a quantity of 0.05 to 20% by weight of active substances relative to the total weight of the cosmetic composition.

12. Cosmetic composition according to any one of the preceding claims, **characterized in that** the surfactants are used in a total quantity of 4% by weight to 30% by weight relative to the total weight of the cosmetic composition.

13. Cosmetic composition according to any one of the preceding claims, **characterized in that** it contains, in addition, a hair-styling polymer.

14. Cosmetic composition according to any one of the preceding claims, **characterized in that** it contains, in addition, a cationic polymer.

15. Method for the cosmetic treatment of keratinous materials, **characterized in that** an effective quantity of a cosmetic composition according to any one of the preceding claims is applied to the keratinous materials, and **in that** rinsing is optionally carried out after an optional leave-in time.

16. Use of a cosmetic composition according to any one of Claims 1 to 14, as a shampoo.

## Patentansprüche

1. Kosmetische Zusammensetzung, **dadurch gekennzeichnet, dass** sie in einem kosmetisch akzeptablen Medium mindestens einen grenzflächenaktiven Stoff, mindestens einen anionischen Latex, mindestens einen Ether mit zwei Fettketten und ein Gemisch von geradkettigen und gesättigten Alkoholen mit langer Kette enthält, das mindestens 50 Gew.-% eines Alkohols enthält, der 22 Kohlenstoffatome aufweist.

2. Kosmetische Zusammensetzung nach Anspruch 1, **dadurch gekennzeichnet, dass** der Ether mit zwei Fettketten unter den Ethern mit zwei Fettketten ausgewählt ist, die bei einer Temperatur von kleiner gleich etwa 30 °C fest sind und der folgenden Formel entsprechen:
R-O-R' (I)
worin bedeuten:
die Gruppen R und R', die gleich oder verschieden sind, eine gesättigte oder ungesättigte, geradkettige oder verzweigte Kohlenwasserstoffgruppe mit 12 bis 30 Kohlenstoffatomen und vorzugsweise 14 bis 24 Kohlenstoffatomen, wobei die Gruppen R und R' so ausgewählt sind, dass die Verbindung der Formel (I) bei einer Temperatur von etwa 30 °C oder darunter fest ist.

3. Kosmetische Zusammensetzung nach Anspruch 1 oder 2, **dadurch gekennzeichnet, dass** der Ether mit zwei Fettketten ein Distearylether ist.

4. Kosmetische Zusammensetzung nach einem der vorhergehenden Ansprüche, **dadurch gekennzeichnet, dass** das Gemisch von Fettalkoholen mindestens 50 Gew.-% eines Alkohols enthält, der 22 Kohlenstoffatome aufweist, bezogen auf das Gesamtgewicht des Gemisches.

5. Kosmetische Zusammensetzung nach einem der vorhergehenden Ansprüche, **dadurch gekennzeichnet, dass** der anionische Latex bei der Polymerisation eines oder mehrerer der folgenden ionogenen Monomere gebildet wird: Acrylsäure, Methacrylsäure, Maleinsäure, Crotonsäure, Itaconsäure, *p*-Styrolsulfonsäure, Vinylsulfonsäure, 2-Methacryloyloxyethylsulfonsäure und 2-Acrylamido-2-methylpropylsulfonsäure, einzeln oder im Gemisch mit verschiedenen Monomeren, wie Styrol, Butadien, Ethylen, Propylen, Isopren, Isobuten, Vinyltoluol, Vinylpropionat, Vinylalkohol, Acrylnitril, Chloropren, Vinylchlorid, Vinylidenchlorid, Vinylacetat, Urethanen, Vinylether, Vinylpyrrolidon, Vinylimidazol, Acrylsäureestern, Methacrylsäureestern, Vinylessigsäureestern, Maleinsäureestern, Crotonsäureestern oder Itaconsäureestern.

6. Kosmetische Zusammensetzung nach einem der vorhergehenden Ansprüche, **dadurch gekennzeichnet, dass** der anionische Latex eine wässerige Dispersion ist, die ein Acrylcopolymer enthält, das aus (a) etwa 35 bis 74 Gew.-% Alkylacrylat, (b) etwa 25 bis 65 Gew.-% Alkylmethacrylat und (c) etwa 1 ist 15 Gew.-% einer oder mehrerer ethylenischer Carbonsäuren oder deren Salzen mit 3 bis 5 Kohlenstoffatomen gebildet ist, wobei die Alkylgruppen 1 bis 5 Kohlenstoffatome enthalten und wobei die prozentualen Gewichtsangaben bezogen auf das Gesamtgewicht des Polymers angegeben sind.

7. Kosmetische Zusammensetzung nach Anspruch 6, **dadurch gekennzeichnet, dass** das Polymer 50 bis 60 Gew.-% Ethylacrylat, 30 bis 40 Gew.-% Methylmethacrylat, 2 bis 10 Gew.-% Acrylsäure und 2 bis 10 Gew.-% Methacrylsäure enthält, wobei die Gesamtkonzentration von Acrylsäure und Methacrylsäure 15 % des Gesamtgewichts des Copolymers nicht übersteigt.

8. Kosmetische Zusammensetzung nach einem der vorhergehenden Ansprüche, **dadurch gekennzeichnet, dass** die grenzflächenaktiven Stoffe unter den anionischen, amphoteren, nichtionischen oder kationischen grenzflächenaktiven Stoffen und deren Gemischen ausgewählt sind.

9. Kosmetische Zusammensetzung nach einem der vorhergehenden Ansprüche, **dadurch gekennzeichnet, dass** der Ether mit zwei Fettketten in einer Menge von 0,5 bis 15 Gew.-%, bezogen auf das Gesamtgewicht der kosmetischen Zusammensetzung, verwendet wird.

10. Kosmetische Zusammensetzung nach einem der vorhergehenden Ansprüche, **dadurch gekennzeichnet, dass** das Gemisch aus geradkettigen und gesättigten Fettalkoholen mit langer Kette, das mindestens 50 Gew.-% eines Alkohols enthält, der 22 Atome aufweist, in einer Menge von 0,5 bis 15 Gew.-%, bezogen auf das Gesamtgewicht der kosmetischen Zusammensetzung, verwendet wird.

11. Kosmetische Zusammensetzung nach einem der vorhergehenden Ansprüche, **dadurch gekennzeichnet, dass** der anionische Latex in einer Menge von 0,05 bis 20 Gew.-% wirksame Substanzen, bezogen auf das Gesamtgewicht der kosmetischen Zusammensetzung, verwendet wird.

12. Kosmetische Zusammensetzung nach einem der vorhergehenden Ansprüche, **dadurch gekennzeichnet, dass** die grenzflächenaktiven Stoffe in einer Gesamtmenge von 4 bis 30 Gew.-%, bezogen auf das Gesamtgewicht der kosmetischen Zusammensetzung, eingesetzt werden.

13. Kosmetische Zusammensetzung nach einem der vorhergehenden Ansprüche, **dadurch gekennzeichnet, dass** sie ferner ein Polymer für die Frisurengestaltung enthält.

14. Kosmetische Zusammensetzung nach einem der vorhergehenden Ansprüche, **dadurch gekennzeichnet, dass** sie ferner ein kationisches Polymer enthält.

15. Verfahren zur kosmetischen Behandlung von Keratinsubstanzen, **dadurch gekennzeichnet, dass** eine wirksame Menge einer kosmetischen Zusammensetzung nach einem der vorhergehenden Ansprüche auf die Keratinfasern aufgetragen wird und **dadurch**, dass nach einer gegebenenfalls abgewarteten Einwirkzeit gegebenenfalls gespült wird.

16. Verwendung einer kosmetischen Zusammensetzung nach einem der Ansprüche 1 bis 14 als Haarwaschmittel.
